(19) Europäisches Patentamt
European Patent Office
Office européen des brevets

(11) EP 4 751 740 A1

(12) **EUROPEAN PATENT APPLICATION**
published in accordance with Art. 153(4) EPC

(43) Date of publication:
**03.06.2026 Bulletin 2026/23**

(21) Application number: **24844931.6**

(22) Date of filing: **24.07.2024**

(51) International Patent Classification (IPC):
*A61K 49/18 (2006.01)* *B82Y 5/00 (2011.01)*
*B82Y 15/00 (2011.01)* *A61K 41/00 (2020.01)*
*A61B 5/055 (2006.01)* *A61N 2/00 (2006.01)*

(86) International application number:
**PCT/ES2024/070474**

(87) International publication number:
**WO 2025/022037 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.07.2023 ES 202330643**

(71) Applicants:
- **Fundación Pública Andaluza Progreso y Salud**
  **41092 Sevilla (ES)**
- **Universidad de Sevilla**
  **41013 Sevilla (ES)**

(72) Inventors:
- **GARCÍA MARTÍN, María Luisa**
  **41092 Sevilla (ES)**
- **CARO SALAZAR, Carlos**
  **41092 Sevilla (ES)**
- **PÁEZ MUÑOZ, José María**
  **41092 Sevilla (ES)**
- **PERNIA LEAL, Manuel**
  **41013 Sevilla (ES)**

(74) Representative: **San Martin Alarcia, Esther**
**C/ Zumaia 33A, 1º b**
**48007 Bilbao (ES)**

(54) **MAGNETIC NANOPARTICLES FUNCTIONALISED WITH CARBOHYDRATES**

(57) The present invention describes metallic magnetic nanoparticles, preferably iron oxide, functionalised with specific ligands, preferably belonging to carbohydrates, having a high capacity for penetration into solid tumours in the central nervous system, specifically high-grade gliomas. This invention further relates to the synthesis method and uses thereof.

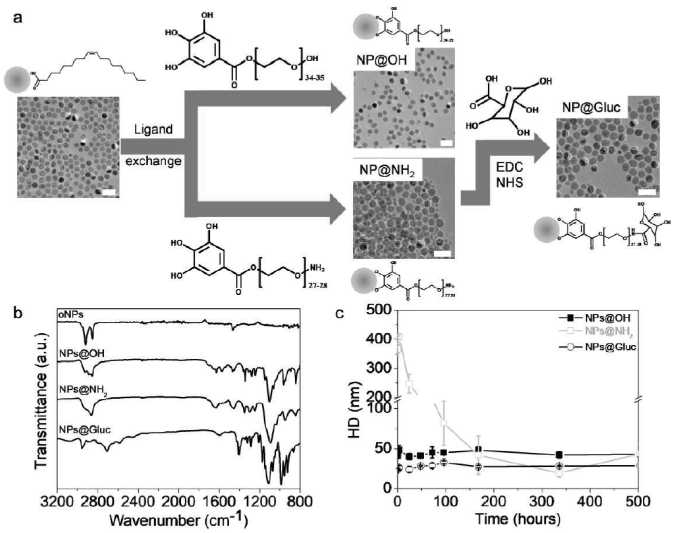


FIG.1



EP 4 751 740 A1

## Description

### FIELD OF THE INVENTION

The invention falls within the field of biotechnology and relates to the development of Metallic Magnetic Nanoparticles (MMNPs), particularly Iron Oxide Nanoparticles (IONPs), with specific ligands functionalised with carbohydrates, for biomedical applications such as the diagnosis and treatment of central nervous system (CNS) tumours.

### PREVIOUS STATE OF THE ART

The World Health Organization (WHO) estimated in 2020 that cancer ranks between the second and fifth leading causes of death worldwide. The most common primary tumours of the central nervous system are gliomas, which are classified from benign tumours (grade I) to very aggressive tumours (grade IV). Glioblastoma multiforme (GBM) is a grade IV glioma, responsible for 15% of all diagnoses of primary malignant CNS tumours, and has a very poor prognosis, with a median survival of only about 15 months, making it one of the most significant challenges in neuro-oncology.

The diagnosis of GBM is based on neuroimaging examinations and histological and molecular information, i.e., on "integrated diagnosis." Magnetic Resonance Imaging (MRI) is the gold-standard technique for evaluating CNS tumours, as it provides excellent morphological and functional images in a non-invasive manner. The use of clinical contrast agents (CAs) is often necessary, but these have limitations due to their toxicity and low specificity.

The standard therapeutic approach includes maximum safe resection, followed by radiotherapy combined with temozolomide and/or temozolomide as an adjuvant. Due to the aggressiveness of GBM, progression is expected in all cases, and when deciding on additional treatment, the patient's condition, the size and location of the tumour, and the time elapsed since the first treatment must be taken into account.

Therefore, there is an urgent need to improve the diagnostic specificity of *in vivo* imaging and, more importantly, the therapeutic efficacy of GBM treatments.

Nanotechnology is dedicated to monitoring, evaluating, shaping, controlling, and manufacturing materials in the size range of 1 to 100 nm, where unique physicochemical phenomena arise that promote novel applications in physics, chemistry, biology, materials science, engineering, and the field of health.

Among nanomaterials, and specifically metallic magnetic nanoparticles (MMNPs) developed to improve cancer diagnosis and treatment, iron oxide nanoparticles (IONPs) have attracted particular interest as they can be used both for cancer diagnosis (CAs for MRI) and treatment (magnetic hyperthermia or drug nanocarriers). In fact, there are several IONPs commercially available on the market (Sinerem, Combidex, Farahem, Feridex, and Endorem) **[1].**

However, none of them is suitable for the diagnosis/treatment of GBM. This is most likely due to the presence of a highly restrictive blood-brain barrier (BBB), which prevents many compounds from reaching their target or doing so with sufficient efficacy. The blood-brain barrier (BBB) has a complex structural and functional composition that makes it almost impermeable, adding an additional challenge to both the diagnosis and treatment of pathological conditions of the CNS, including brain tumours **[2].**

In the case of IONPs (or any other type of nanoparticles - NPs), the Enhanced Permeability and Retention (EPR) effect has long been considered the most efficient mechanism for their accumulation in solid tumours. This is a passive transport mechanism based on the size of the NPs, which is theoretically optimal for NPs ranging from 20 to 100 nm **[3,4].**

To date, this passive transport mechanism has also been widely relied upon to deliver NPs across the BBB.

However, as has recently been demonstrated, the EPR effect is extremely inefficient in brain tumours, especially in the case of metallic NPs, due to the restrictions imposed by the blood-brain tumour barrier (BBTB) **[5].**

NanoTherm® (MAGFORCE) has developed the first and only nanotechnology-based therapy with European approval for the treatment of brain tumours, whose IONPs are introduced directly into the tumour or the wall of the resection cavity by stereotactic administration **[6].** In other words, they do not use intravenous administration, although a more homogeneous distribution of NPs in the tumour interstitium can be expected using this route of administration, especially in the well-vascularised (more active) parts of the tumour. The choice of intratumoural administration is therefore clearly related to the ineffective accumulation of IONPs by passive transport via the EPR effect after intravenous administration.

Glucose transporters (GLUT) in the BBB maintain the continuously high demands for glucose of the CNS, as this is almost entirely dependent on glucose as an energy source. Some GLUTs (mainly GLUT1) have been found to be significantly overexpressed in patients with GBM **[7-9],** and GLUT1, particularly in the vascular endothelium of GBM tumours **[10].**

Therefore, overexpression of GLUT1 in the tumour vasculature would, in principle, allow NPs functionalised with glucose derivatives to be transported and accumulated in the GBM (active tumour targeting).

This strategy to facilitate the passage of various compounds through the BBB has been studied in healthy brains, using lipid nanoparticles functionalised with the mannose derivative, p-aminophenyl-$\alpha$-d-mannopyranoside (MAN) **[11].** However, the contribution of NP functionalisation with mannose to its ability to cross the BBB is questionable, as this may

be largely due to the lipid nature of the NP, since lipophilicity is considered the key factor for better penetration into the brain.

It should also be considered that in the case of brain tumours, the properties of the BBB are modified, so it is not possible to extrapolate the behaviour that these NPs would have when crossing the BBTB. Therefore, to date, there are no reports describing NPs of any composition capable of crossing the BBTB.

Furthermore, although GLUT1 is overexpressed in the BBTB compared to the BBB of healthy patients, under *in vivo* conditions, its affinity for glucose substantially exceeds its affinity for any other glucose derivative **[12],** so NPs functionalised with glucose derivatives must compete with naturally occurring glucose, which reduces their ability to be transported across the BBTB.

## DESCRIPTION OF THE INVENTION

The present invention describes IONPs functionalised with specific ligands belonging to the carbohydrate family that have a greater ability to penetrate solid CNS tumours, specifically high-grade gliomas, particularly glioblastoma multiforme (GBM), thanks to the specific recognition of the carbohydrate by specific receptors/transporters, mainly glucose membrane transporters (GLUTs), which allow them to cross the blood-brain tumour barrier (BBTB).

IONPs are functionalised with carbohydrates, preferably glucose derivatives, and more preferably, glucuronic acid.

Since the facilitated transport of glucose and derivatives by GLUT1 is saturable, the decrease in circulating glucose levels, such as that produced by a 24-hour fasting period, allows for a further increase in the transport of NPs functionalised with a glucose derivative through the vascular endothelium, in this case with glucuronic acid, by reducing the competition with glucose for GLUT1.

Therefore, combined with mild hypoglycaemia, glucuronic acid-functionalised IONPs are actively targeted through BBTB to high-grade gliomas, and since GLUT1 and GLUT3 are also often overexpressed in tumour cells, particularly in GBM stem cells (GSCs) **[13],** IONPs functionalised in this way are also suitable for active targeting of GSCs. In other words, this active targeting is capable of reaching two targets: the tumour vascular endothelium, allowing active transport into the tumour interior; and tumour cells, particularly GSCs.

Thus, they are not only capable of crossing the BBTB, but also of internalizing themselves in tumour cells through GLUT1, thus constituting an optimal therapeutic nanoplatform capable of reaching not only the tumour interstitium, but also tumour cells, especially GSCs, which are largely responsible for the resistance of GBMs to chemotherapy and radiotherapy **[14].**

Once accumulated in the tumour, these functionalised IONPs can be used as theranostic nanosystems, i.e., for diagnostic purposes, where IONPs act as contrast agents for Magnetic Resonance Imaging (MRI), and for therapeutic purposes, where IONPs act as mediators of magnetic hyperthermia or as platforms for drug transport and delivery. This theranostic approach falls into the category of "see-treat-see" strategies, as the theranostic nanosystem is monitored in real time using MRI.

They are therefore much more effective, both for diagnostic and therapeutic use, than other types of NPs. This ability to cross the vascular endothelial barrier of the glioma and accumulate specifically in the tumour has not been achieved previously by any other known NP or compound.

In general terms, the present invention provides nanoparticles or nanostructures, composed mainly of iron/iron oxides or derivatives, which have polymeric ligands on their surface to improve their colloidal stability, as well as to promote subsequent functionalisations. Subsequent functionalisation with carbohydrates, mainly derived from glucose, such as glucuronic acid, but not exclusively, facilitates the specific recognition of certain molecular targets (glucose transporters or GLUTs), allowing the successful accumulation of NPs in solid tumours, specifically in high-grade gliomas, this accumulation being greatly enhanced by mild hypoglycaemia, such as that produced by a 24-hour fasting period.

IONPs or their derivatives are of a suitable size to avoid ferromagnetic behaviour, which would limit their *in vivo* applications.

NPs/nanostructures functionalised with carbohydrates of biomedical interest can in turn be used as neo-nanoplatforms, as they allow the anchoring of other types of biomolecules, thus increasing their versatility.

The NPs covered by this invention have numerous advantages over existing ones. Mainly, their accumulation in solid tumours or metastatic lesions increases in short periods of time due to their active targeting, which results in a higher probability of reaching a therapeutic dose within these tumours.

The present invention also describes the procedure for the preparation of new MRI CAs based on IONPs, or their derivatives, on whose surface organic ligands derived from polymers and functionalised with carbohydrates are strongly anchored. This procedure includes the following steps:

1. Synthesis of the metallic magnetic core from metallic precursors, preferably an iron precursor, and in particular iron oleate, by means of a chemical or physical process, such as thermal decomposition. Regarding thermal decomposition, a critical point is an extreme control over the ramp temperature.

2. Functionalisation of the metallic magnetic core with polymeric ligands using a physical or chemical method. The polymeric ligands, capable of stabilizing the metallic magnetic core in aqueous media, are synthesized by a covalent bond between a catechol and a polymer. Preferably, the anchor molecule is gallic acid (GA), and the polymer is bis-amino polyethylene glycol $NH_2$-PEG-$NH_2$. As an example, IONPs suspended in toluene are incubated with a GA-$PEG_{2000}$-$NH_2$ ligand for 1 hour in an ultrasonic bath and then for 4 hours in a temperature-controlled water bath. Preferably, the temperature is in the range of 20-80°C, more preferably between 40 and 60°C.

3. Final functionalisation with a compound belonging to the carbohydrate family (mainly glucuronic acid, but not exclusively) using a physical or chemical method. Final functionalisation with the carbohydrate gives the nanoparticles the ability to specifically recognize cell receptors/transporters, enabling active targeting.

At each step, the nanoparticles are purified by dialysis against Milli-Q water or by centrifugation using suitable filters.

In order to test the IONPs of the invention, *in vivo* experiments were performed in mice as animal model. Pharmacokinetics and biodistribution were analyzed *in vivo* using MRI. Tumours were implanted orthotopically in the rodents. High-grade glioma cells were implanted stereotactically in the right caudate nucleus. Once the tumour reached the appropriate size, the metallic magnetic nanoparticles of the present invention were administered intravenously to 24h fasted animals. Dynamic and parametric MRI sequences were used for short- and long-term monitoring of the injected NPs *(in vivo* pharmacokinetics).

It was concluded that the IONPs of the invention have very high stability (more than 500 hours), high internalization capacity, are not cytotoxic, and are biocompatible. They also have an excellent ability to remain in the blood and to accumulate selectively in tumour tissue, with this accumulation being promoted under conditions of mild hypoglycemia. Furthermore, once accumulated in the tumour, the IONPs of the invention were distributed homogeneously throughout the tumour tissue, which is also crucial for their efficacy.

Therefore, a **first aspect** of the invention relates to a **metallic magnetic nanoparticle,** hereinafter referred to as the "metallic magnetic nanoparticle of the invention," which comprises at least one organic ligand attached to its surface, characterised in that it is functionalised by chemical bond with at least one molecule belonging to the carbohydrate family.

In a preferred embodiment, the metal core is composed of iron or M-iron, where M is selected from the list comprising cobalt, manganese, zinc, nickel, gold, silver, platinum, palladium, and ruthenium, either in metallic form or as an oxide, and more preferably iron oxide. Preferably, its metal core has a size between 1 and 100 nanometres.

In another preferred embodiment, the organic ligand attached to the surface of the nanoparticle is a catechol derivative, such as L-DOPA, dopamine, caffeic acid, dihydrocaffeic acid, epinephrine, norepinephrine, noradrenaline, adrenaline, protocatechuic acid, and gallic acid, preferably gallic acid, covalently bound to a polymer selected from the list comprising polyethylene glycol, polyvinyl alcohol, polyvinylpyrrolidone, polyacrylic acid, chitosan, and derivatives thereof. Preferably, the catechol derivative is gallic acid and the polymer is polyethylene glycol or derivatives thereof, and more preferably bis-amino polyethylene glycol. More preferably, the polyethylene glycol has a molecular weight in the range of 200-6000 kDa and even more preferably between 1000 and 3000 kDa.

In another preferred embodiment, the carbohydrate is selected from the list comprising allose, altrose, glucose, gulose, mannose, idose, galactose, trehalose, and derivatives thereof. Preferably, the carbohydrate is a hexose, more preferably glucose or derivatives thereof, and most preferably glucuronic acid.

In a particularly preferred embodiment of the invention, the metallic magnetic nanoparticle has a metallic core composed of iron oxide, comprises at least one organic ligand derived from catechol, specifically gallic acid, covalently bound to a polyethylene glycol derivative, preferably bis-amino polyethylene glycol, fixed to the surface, and is characterised in that it is functionalised with a glucose derivative, preferably glucuronic acid.

Preferably, the metallic magnetic nanoparticle of the invention is suitable for intratumoural, intranasal, intraperitoneal, intravenous, intraarterial, anal, or oral administration.

**A second aspect of the invention** relates to **the method of synthesizing the** metallic magnetic **nanoparticle** of the invention, hereinafter referred to as the "synthesis method of the invention," which comprises the following steps:

a) Synthesis of the metal core from at least one metal precursor selected from the list comprising chloride, sulfate, nitrate, carbonyl, acetylacetonate, iron oleate, and their derivatives, using a chemical or physical process selected from the list comprising evaporation, condensation, laser ablation, hydrothermal method, chemical reduction, irradiation, coprecipitation, electrochemistry, microemulsion, thermal decomposition, and photoreduction.

b) Functionalisation with a polymeric ligand synthesized by a covalent bond between a catechol derivative selected from the list comprising gallic acid and a polymer selected from the list comprising polyethylene glycol, polyvinyl alcohol, polyvinylpyrrolidone, polyacrylic acid, chitosan, or derivatives thereof, by a physical or chemical method.

c) Functionalisation with a compound belonging to the carbohydrate family is selected from the list comprising allose, altrose, glucose, gulose, mannose, idose, galactose, trehalose, or derivatives thereof, such as glucuronic acid.

In a preferred embodiment of the synthesis method of the invention, the synthesis of the metal core is carried out by thermal decomposition. In a more preferred embodiment, the decomposition is carried out in an organic solvent, at a high temperature, preferably in the range 180-350°C and in an inert atmosphere. Preferably, the solvent for the reaction is an organic compound, more preferably 1-octadecene. In an even more preferred embodiment, stabilizing agents are used for the reaction, and preferably these are selected from oleic acid, oleyl alcohol, oleylamine, and 1,2-hexadecanethiol and mixtures thereof.

In another preferred embodiment, the synthesis of the metal core is carried out from more than one metal precursor. In another preferred embodiment, the metal precursor is iron oleate.

In a preferred embodiment, the first functionalisation is performed with the polymeric ligand GA-PEG-$NH_2$.

In another preferred embodiment, said functionalisation is carried out in two steps: a first step by applying ultrasound and a second step in which the reaction temperature is increased.

In a preferred embodiment, the second functionalisation is carried out with glucuronic acid.

In another preferred embodiment, the link with carbohydrates is carried out using carbodiimide as the coupling agent, preferably 1-ethyl-3-(3-dimethylaminopropyl)carbodiimide in the presence of N-hydroxysuccinimide.

Preferably, in each step of the synthesis method of the invention, the nanoparticles are purified by dialysis against Milli-Q water or by centrifugation using suitable filters.

A **third aspect of the invention** relates to **the metallic magnetic nanoparticle** of the invention **for its use** in a method for the diagnosis, evaluation, acquisition of images, or treatment of solid tumours or metastatic lesions, preferably of the central nervous system.

In another embodiment of this aspect of the invention, the metallic magnetic nanoparticle of the invention is described for use as a contrast agent for Magnetic Resonance Imaging, preferably for the evaluation of solid tumours or metastatic lesions, and more preferably for tumours of the central nervous system.

In another embodiment of this aspect of the invention, the metallic magnetic nanoparticle of the invention is described for its use as a drug delivery platform, preferably for drugs targeting the treatment of solid tumours or metastatic lesions, and more preferably for tumours of the central nervous system.

In another embodiment of this aspect of the invention, the metallic magnetic nanoparticle of the invention is described for its use as a mediator of magnetic hyperthermia, preferably directed to the treatment of solid tumours or metastatic lesions, and more preferably, tumours of the central nervous system.

In another embodiment of this aspect of the invention, the metallic magnetic nanoparticle of the invention is described for its use via intratumoural, intranasal, intraperitoneal, intravenous, intraarterial, anal, or oral administration.

In another embodiment of this aspect of the invention, the metallic magnetic nanoparticle of the invention is described for its use in patients who are in a state of mild hypoglycemia or near the lower limit of preprandial normoglycemia.

Preferably, said state is derived from fasting or is due to prior administration of a chemical compound selected from the list comprising Insulin, Biguanides, Sulfonylureas, Glitinides, Dipeptidyl Peptidase IV (DPP-IV) Inhibitors, Sodium-Glucose Cotransporter-2 (SGLT2) Inhibitors, Glucagon-Like Peptide-1 (GLP-1) Agonists, Alpha-Glucosidase Inhibitors (AGIs), Thiazolidinediones, or (SGLT2) inhibitors, glucagon-like peptide-1 (GLP-1) agonists, alpha-glucosidase inhibitors (AGIs), thiazolidinediones, or related compounds.

## DETAILED DESCRIPTION OF THE FIGURES

**Figure 1. a)** Diagram of the different functionalisation steps, showing the chemical structure of the different PEGylated ligands used and glucuronic acid. The size and shape of the IONPs throughout the process were evaluated by electron microscopy (representative images are shown). The scale corresponds to 50 nm in all cases. **b)** Evaluation by Fourier Transform Infrared (FTIR) of the different functionalisation steps. **c)** Measurement of the hydrodynamic diameter of the different functionalised IONPs over time.

**Figure 2.** Transverse relaxations ($r_2$) calculated as the slope of the fitting line of R2 versus iron concentration, calculated at different magnetic fields: 1.44 T (blue) and 9.4 T (black) for NPs@OH **(a)** and NPs@Gluc **(b).** High-field T2-weighted images corresponding to each concentration are shown on the right margin.

**Figure 3.** Representative optical microscopy images of HFF-1 fibroblasts resulting from the merge of bright field (grey), Hoechst 33342 (blue), and TOPRO-3 Iodine (red) images: **(a)** negative control, **(b)** positive control, **(c)** cells

exposed to 100 μg/mL of NPs@OH NPs. Scale bar corresponds to 50 μm. **(d)** Total number of cells per well after exposure to increasing concentrations of NPs@OH NPs. **(e)** Percentage of dead cells after exposure to increasing concentrations of NPs@OH NPs. **(f)** MTT assay of cells exposed to increasing concentrations of NPs@OH NPs.

**Figure 4.** Representative optical microscopy images of HFF-1 fibroblasts resulting from the fusion of bright field (grey), Hoechst 33342 (blue), and TOPRO-3 Iodine (red): **(a)** negative control, **(b)** positive control, **(c)** cells exposed to 100 μg/mL of NPs@Gluc NPs. Scale bar corresponds to 50 μm. **(d)** Total number of cells per well after exposure to increasing concentrations of NPs@Gluc NPs. **(e)** Percentage of dead cells after exposure to increasing concentrations of NPs@Gluc NPs. **(f)** MTT assay of cells exposed to increasing concentrations of NPs@Gluc NPs.

**Figure 5.** Representative optical images of HFF1, C6, u87, and 4T1 cells exposed to NPs@OH (left) and NPs@Gluc (right), both at an iron concentration of 100 μg/mL, for 2 hours and 4 hours, both NPs stained with Prussian blue. Cell uptake is only observed in NPs functionalised with glucuronic acid.

**Figure 6. a)** Western blot analysis of the expression of specific glucose transporters (GLUT1 and GLUT3) in C6 cells versus MW - Molecular Weight. Glucose competition experiment: **b)** Representative optical images of C6 cells exposed to different glucose concentrations and a constant concentration of 100 μg/mL of NPs@Gluc stained with Prussian blue, and c) quantitative evaluation by Inductively Coupled Plasma High-Resolution Mass Spectrometry (ICP-HRMS).

**Figure 7. a)** Short-term pharmacokinetics obtained by dynamic MRI over the whole tumour (red) and in the tumour periphery (blue) during the first 30 minutes after intravenous administration of NPs@OH (G1), NPs@Gluc (G2), and NPs@Gluc in mice subjected to overnight fasting (G3). **b)** T2-weighted MR images at 1h and 2h after administration of the NPs, from the different experimental groups, and the corresponding quantitative analysis by calculating the relaxation rate variation ($\Delta R_2$) over time over the whole tumour **(c)** and the tumour periphery **(d).**

**Figure 8. a)** Representative electron microscopy images of blood 1 hour after injection of NPs@OH (top) and NPs@Gluc (bottom) **b)** Variation in relaxation rate ($\Delta R_2$) of plasma samples 1 hour after intravenous injection of NPs@OH (grey) and NPs@Gluc (blue) corrected with normal plasma values.

**Figure 9.** Representative histological sections of Prussian blue staining of liver and tumours 2 hours post-injection of PBS **(a),** NPs@Oh **(b)** NPs@Gluc. The scale bar corresponds to 50 μm.

## EXAMPLE OF THE INVENTION

### Synthesis of iron oxide nanoparticles (IONPs), their functionalisation with PEGylated ligands and finally with glucose derivatives.

Synthesis of iron oleate (iron precursor)

A mixture of 10.8 g of iron chloride (40 mmol) and 36.5 g of sodium oleate (120 mmol) was dissolved in 80 mL of ethanol, 60 mL of distilled water, and 140 mL of hexane. The resulting solution was heated to 70°C and left for 4 hours in an inert atmosphere, allowing the hexane to reflux. Then, the reaction was cooled to room temperature and two phases were distinguished: a lower aqueous phase and an upper organic phase containing the iron oleate. The organic phase was washed 3 times with distilled water, and the hexane was evaporated in a rotavapor.

Synthesis of IONPs by thermal decomposition

1.81 g (5.3 mmol) of iron oleate and 0.32 g (1.133 mmol) of oleic acid were weighed into a double-necked round-bottom flask with a maximum volume of 50 mL. 15 mL of 1-octadecene was used as the solvent. The reaction was then preheated to 200°C under an inert atmosphere ($N_2$) and magnetic stirring. The temperature was increased to 320°C using a ramp temperature of 1°C/min. The temperature of 320°C was maintained for 1 hour and then allowed to cool to room temperature. The NP suspension was washed 3 times with a mixture of acetone:ethanol (1:1), centrifuged at 8000 rpm for 20 minutes to remove unwanted reagents, and finally resuspended in toluene. The nanoparticle suspension was kept at 4°C.

Synthesis of PEGylated ligands

Synthesis of GA-PEG-OH with formula:

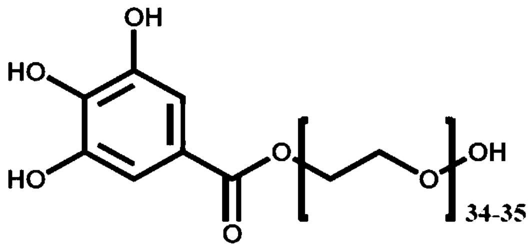

Briefly, to a solution of polyethylene glycol (Molecular weight: 3000 g/mol, 1 mmol, 3.0 g), gallic acid (Molecular weight: 170 g/mol, 1 mmol, 170 mg), and 4-(dimethylamino) pyridine (Molecular weight: 122 g/mol, 200 μmol, 24 mg) in 100 mL of tetrahydrofuran and 10 mL of dichloromethane, in a round-bottom flask under a nitrogen atmosphere, a solution of dicyclohexyl carbodiimide (Molecular weight: 206 g/mol, 5 mmol, 1 g) in tetrahydrofuran was added dropwise. The mixture was stirred overnight at room temperature. The reaction mixture was filtered through filter a paper and the solvents were evaporated by rotation.

Synthesis of GA-PEG-$NH_2$ with formula:

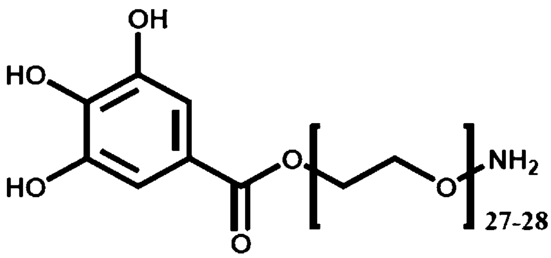

Briefly, to a solution of α,ω-Bis-amino polyethylene glycol (Mw: 2000 g/mol, 1 mmol, 2.0 g), gallic acid (Mw: 170 g/mol, 1 mmol, 170 mg), and 4-(dimethylamino) pyridine (Mw: 122 g/mol, 200 μmol, 24 mg) in 100 mL of tetrahydrofuran and 10 mL of dichloromethane, in a round-bottom flask under a nitrogen atmosphere, a solution of dicyclohexyl carbodiimide (Mw: 206 g/mol, 5 mmol, 1 g) in tetrahydrofuran was added dropwise. The mixture was stirred overnight at room temperature. The reaction mixture was filtered through a filter paper and the solvents were evaporated by rotation

Ligand exchange process.

Briefly, a solution containing 1.0 mL of NPs (10 g/L Fe), 1.0 mL of the GA-PEG-OH ligand at a concentration of 0.1 M in $CHCl_3$, and 50 μL of triethylamine was added to a glass vial. The mixture was ultrasonicated for 1 h and kept at 55°C for 4 h. At this point, the mixture was diluted with 5 mL of toluene, 5 mL of milli-Q water, and 10 mL of acetone. Then, it was shaken to transfer the functionalised NPs to the aqueous phase. The aqueous phase was then collected in a round-bottom flask and the residual organic solvents were evaporated by rotation. The PEGylated NPs were then purified on centrifuge filters with a molecular weight cut-off of 100 kDa at 450 rcf. After each centrifugation, the functionalised NPs were resuspended with milli-Q water. The purification step was repeated several times until the filtered solution was clear. After purification, the PEGylated NPs were resuspended in PBS buffer. Finally, to ensure high stability of the mono-dispersed NPs, this solution was centrifuged at 150 rcf for 5 min and placed on a permanent magnet (0.6 T) for 5 min. These nanoparticles will hereinafter be referred to as NPs@OH.

Functionalisation with GA_PEG-$NH_2$ was performed in a similar manner.

These nanoparticles will hereinafter be referred to as NPs@$NH_2$.

Covalent reaction with carbohydrates

In a round-bottom flask, 1 mL of NPs@$NH_2$ (0.6-0.7 mg/mL of iron), 0.192 mg (1.67 μmol) of NHS (N-hydroxysuccinimide), 0.192 mg (1.24 μmol) of EDC (1-ethyl-3-3-dimethylaminopropylcarbodiimide), and 0.162 mg (0.834 μmol) of glucuronic acid were added. The reaction was carried out on ice. After 2 hours of reaction, the mixture was purified on centrifuge filters with a molecular weight cut-off of 100 kDa at 450 rcf to remove unreacted reagents. These nanoparticles will hereinafter be referred to as NPs@GLUC.

Characterisation of NPs by Inductively Coupled Plasma High-Resolution Mass Spectrometry (ICP-HRMS).

The Fe concentration was determined on a NexION ICP-HRMS (Perkin-Elmer, Waltham, MA, U.S.A). Briefly, 2.5 mL of aqua regia was added to 25 μL of a nanoparticle solution in a volumetric flask. The mixture was left overnight. Then Milli-Q water was added to complete the total volume of 25 mL.

Characterisation of NPs by Transmission Electron Microscopy (TEM).

TEM images were obtained using a FEI Tecnai G2 Twin microscope operated at an acceleration voltage of 100 kV. TEM samples were prepared by dropping a solution of nanoparticles corresponding to ~1 g/L of (Fe) onto a carbon-coated copper grid and allowing the solvent to evaporate. The diameters were calculated from an average of one hundred measured nanoparticles.

Characterisation of NPs by Fourier Transform Infra-Red Spectroscopy (FTIR).

FTIR spectra were recorded with a FTIR-4100 Jasco using a single reflection ATR accessory (MIRacle ATR, PIKE Technologies) coupled to a liquid nitrogen-cooled mercury cadmium telluride (MCT) detector. All spectra were recorded in the range of 4000 to 800 $cm^{-1}$ with a resolution of 4 $cm^{-1}$, accumulating 200 scans. All samples were recorded as a solid product.

Characterisation of NPs by Dynamic Light Scattering (DLS).

Measurements of the size distribution of PEGylated IONPs were performed on a Zetasizer Nano ZS90 (Malvern, USA). The nanoparticles were dispersed in milli-Q water or PBS at a concentration of 100 mg/L of Fe. Measurements were performed on a ZEN0118 low-volume disposable sizing cuvette, setting 2,420 as the refractive index and 90° as the detection angle. The measurement duration was set as automatic, and three as the number of measurements. The general purpose was chosen as the analysis model (normal resolution).

Characterisation of NPs by transverse relaxivity (r2).

The transverse relaxation time (T2) was measured by proton Nuclear Magnetic Resonance $^1$H NMR, at low (1.44 T) and high (9.4 T) magnetic fields, using solutions of magnetic NPs in PBS with Fe concentrations ranging from 0.125 to 2 mM under physiological conditions at 37°C. $T_2$ measurements at low magnetic field were performed on a Bruker Minispec system (Bruker BioSpin, Rheinstetten, Germany) using the Carl-Purcell-Meiboom-Gill (CPMG) sequence. At high field, $T_2$ values were measured in a Bruker Biospec MRI system (Bruker Biospec, Bruker BioSpin, Ettlingen, Germany) equipped with 400 mT•$m^{-1}$ field gradients and a 40 mm quadrature "bird-cage" type resonator at 298 K. $T_2$ values were measured using a 64-echo Carl-Purcell-Meiboom-Gill (CPMG) imaging sequence (TE values from 7.5 ms to 640 ms). Transverse relaxivity, $r_2$, in both magnetic fields was calculated from the slope of the linear fit of the relaxation rate ($1/T_2$) versus the Fe concentration, as indicated in the following equation:

$$\frac{1}{T_2} = \frac{1}{T_2(0)} + r_2[Fe]$$

Regions of interest (ROI) were drawn on the first image of the image sequence, and the intensity values were extracted and fitted to the following equations:

$$M_Z(t) = M_0(1 - e^{-TR/T_1})$$

$$M_{XY}(t) = M_0 e^{-TE/T_2}$$

where $M_z$ and $M_{xy}$ are the signal intensities at time TR or TE, and $M_0$ is the signal intensity at equilibrium.

***In vitro* tests.**

Various tests were performed for a comprehensive analysis of the cytotoxicity of IONPs.

A) Cell cultures.

The HFF-1 (human fibroblasts) cell lines were selected as working models and used as a representative control for healthy cells, along with C6 (rat glioblastoma), u87 (human glioblastoma), and 4T1 (mouse breast cancer) cell lines. In all cases, standard culture conditions were used, including the use of three different culture media: Dulbecco's Modified Eagle's (DMEM) for HFF-1 and C6 cells; Roswell Park Memorial Institute (RPMI) for 4T1 cells; DMEM/F12 for u87 cells. All media were supplemented with 5% serum, penicillin/streptomycin, and L-glutamine. Cells were maintained at 37°C and in an atmosphere containing 5% $CO_2$. Cells were subcultured every 2 days using trypsin.

B) Evaluation of cellular uptake by Prussian blue staining.

$3x10^5$ cells were seeded in a 24-well plate at 37°C in a 5% $CO_2$ atmosphere. After 24 hours, the medium was replaced with fresh medium containing PEGylated IONPs. The cells were incubated again for 2 h and 4 h. After incubation with the NPs, the cells were washed three times with PBS to remove free NPs. Later, cells were fixed for 40 minutes using 4% paraformaldehyde (Sigma-Aldrich). Then, cells were washed three times with PBS and incubated with freshly prepared Prussian blue (4% potassium ferrocyanide [Sigma-Aldrich]/12% HCl, 1:1, v/v) for 30 minutes. The cells were washed three times with PBS, stained with "nuclear fast red" (Sigma-Aldrich), and observed with an inverted optical microscope.

C) Western blot.

First, the protein extract was prepared by cell lysis. For this purpose, the cells were cultured to confluence. The cells were then washed three times with PBS at 4°C and lysis buffer was added. Later, the cell suspension was transferred and centrifuged in a precooled centrifuge tube. Proteins remained in the supernatant. Once the extract was obtained, the proteins were separated by gel electrophoresis according to their molecular weight. The gel was transferred to the membrane for immunodetection. Anti-GLUT1 and anti-GLUT3 antibodies were used for this work.

D) Quantification of cellular uptake by Inductively Coupled Plasma High-Resolution Mass Spectrometry (ICP-MS).

Similarly, as mentioned above, $1x10^5$ 4T1 cells were seeded in a 96-well plate at 37°C in an atmosphere with 5% $CO_2$. After 24 h, the medium was replaced with a new one containing PEGylated IONPs. Cells were incubated again for 2 h and 4 h. After incubation with NPs, the cells were washed three times with PBS to remove any free NPs. At that point, 100 $\mu$l of aqua regia was added to each well. The mixture was left overnight. The, milli-Q water was added to complete the total volume to 10 mL. Fe concentration was determined on a NexION ICP-HRMS (Perkin-Elmer, Waltham, MA, USA).

E) Glucose competition assays.

To demonstrate the expected transport of NPs@Gluc through glucose transporters (GLUTs), we performed a glucose competition assay. For this assay, C6 cells were grown to confluence and incubated with a constant concentration of NPs@Gluc [100 $\mu$g/mL] while increasing the glucose concentration. After 2 hours, cells were stained with Prussian blue, as described in section B). In addition, quantitative analysis by ICP-MS was also performed, as described in section D).

F) High Content Screening (HCS).

HFF-1 cells were seeded at a density of $1x10^4$ cells/well in a 96-well plate at 37°C in a 5% $CO_2$ atmosphere (100 $\mu$L per well, number of replicates = 5). After 24 h of culture, the medium in the wells was replaced with fresh medium containing NPs at varying concentrations from 0.1 to 100 $\mu$g/mL (Fe). After 24 h, Triton X-100 was added to the positive control wells. All wells were stained after 15 min with: 1) DAPI (4',6-diamidino-2-phenylindole, dilution 1:3000 in PBS) to label cell nuclei, although with stronger labelling in live cells; 2) calcein (1:1000 in PBS) to assess cell activity; and 3) TO-PRO-3 iodine to label dead cells (1:1000 dilution in PBS). Images of cell morphology were taken with a Perkin Elmer Operetta High Content Imaging System with a 20× LWD 0.45 NA air objective lens. Five well replicates were analyzed for each condition with 10 random image fields captured per well. For each field, fluorescence images were captured for DAPI, Calcein-AM, and TO-PRO-3, in addition to a bright field image. Cell mortality percentages were calculated automatically using Operetta Harmony software, which identified all nuclei (live and dead) from DAPI staining and determined the percentage of dead cells from the number of nuclei showing high levels of TO-PRO-3 staining. Intracellular esterase activity was assessed by Calcein-AM staining.

G) MTT assay.

In summary, HFF-1 cells were seeded at a density of $1x10^4$ cells/well in a 96-well plate at 37°C in a 5% $CO_2$ atmosphere (200 μL per well, number of replicates = 5). After 24 h of culture, the medium in the wells was replaced with fresh medium containing PEGylated IONPs at varying concentrations from 0.1 μg/mL to 100 μg/mL. After 24 h, Triton X-100 was added to the positive control wells and, after waiting 15 min, the supernatant from each well was replaced with 200 μL of fresh medium containing 3-[4,5-dimethylthiazol-2-yl]-2,5-diphenyl tetrazolium bromide (MTT) (0.5 mg/mL). After 2 h of incubation at 37°C and 5% $CO_2$, the medium was removed, the formazan crystals were solubilised with 200 μL of DMSO, and the solution was vigorously mixed to dissolve the reacted dye. The absorbance of each well $[Abs]_{well}$ was read in a microplate reader (Dynatech MR7000 instruments) at 550 nm. Relative cell viability (%) and its error in relation to the control wells containing cell culture medium without nanoparticles were calculated using the equations:

$$RCV(\%) = \left(\frac{[Abs]_{test} - [Abs]_{Pos.Ctrl.}}{[Abs]_{Neg.Ctrl.} - [Abs]_{Pos.Ctrl.}}\right) \times 100$$

$$Error(\%) = RCV_{test} \times \sqrt{\left(\frac{\sigma_{test}}{[Abs]_{test}}\right)^2 + \left(\frac{\sigma_{Ctrl}}{[Abs]_{Ctrl.}}\right)^2}$$

where σ is the standard deviation.

***In vivo* assays.**

In an orthotopic GBM model (mice model), the pharmacokinetics and biodistribution after intravenous administration of PEGylated IONPs were determined using dynamic and parametric MRI.

Animals.

Six-week-old male Balb/c mice (20 g in weight) provided by Charles River (n = 4 per experimental group) were used for the *in vivo* experiments. These experiments were conducted in accordance with the ethical guidelines of the local ethics committee and also in line with national (R.D. 53/2013) and European (2010/62/EU) regulations.

Tumour implantation.

C6 cells were brought to 70-80% confluence. At that point, they were trypsinized and the cells were counted. $10^5$ cells, suspended in 5 μL of culture medium, were inoculated into the right caudate nucleus of mice. Then, the tumours were allowed to grow until they reached a volume of between 0.16 and 0.2 $mm^3$. At this point, the mice were randomly divided into three groups:

G1. Intravenous administration of NPs@OH.

G2. Intravenous administration of NPs@Gluc.

G3. Mice fasted overnight and NPs@Gluc was administered intravenously.

Evaluation by Magnetic Resonance Imaging (MRI).

MRI experiments were performed on a 9.4 T Bruker Biospec system equipped with 400 mT/m gradients and a mouse brain surface antenna for signal reception and a volume antenna for transmission. ParaVision 6.0 software (Bruker Medical GmbH®) was used in the experiments on a Linux platform. Animals were anesthetized with 1% isoflurane, cannulated in the tail vein, and placed in the spectrometer, where respiration and body temperature were monitored throughout the MRI experiment using a Small Animal Monitoring system (PC-SAM 32 v8.02 software). PEGylated IONPs were administered intravenously through the tail vein at a concentration of 10 mg (Fe) per kg.

Pharmacokinetic and biodistribution experiments were performed using the following experimental scheme:

1. acquisition of high-resolution $T_2$-weighted images (0 h),
2. acquisition of susceptibility-weighted images (0 h),
3. acquisition of $T_2$ parametric images (quantitative) (0 h),
4. intravenous injection of PEGylated IONPs,
5. acquisition of a dynamic sequence of $T_2$-weighted images (pharmacokinetic),
6. acquisition of high-resolution $T_2$-weighted images (30 min),
7. acquisition of susceptibility-weighted images (30 min),
8. acquisition of parametric $T_2$ (quantitative) images (30 min),
9. acquisition of high-resolution $T_2$-weighted images (1 h),
10. acquisition of susceptibility-weighted images (1 hour),
11. acquisition of parametric $T_2$ (quantitative) images (1 h),
12. acquisition of high-resolution $T_2$-weighted images (2 h),
13. acquisition of susceptibility-weighted images (2 h),
14. acquisition of parametric $T_2$ (quantitative) images (2 h).

Susceptibility-weighted images were acquired using a FLASH sequence (TE = 16 ms, TR = 350 ms, 4 averages, 1 repetition, flip angle = 40°, FOV = 1 cm, matrix size = 512 x 256).

High-resolution $T_2$-weighted images were acquired using a turbo-RARE sequence with respiratory synchronization (TE = 27 ms, TR = 1000 ms, 4 averages, 156 μm in-plane resolution, and 1 mm slice thickness).

Quantitative $T_2$ measurements were also performed using a multi-echo spin-echo CPMG sequence (TE from 7 ms to 448 ms, TR = 3500 ms, FOV = 4 cm, matrix size = 128x128, slice thickness = 1 mm). For short-time pharmacokinetics based on $T_2$ images, the turbo-RARE sequence was used with the same parameters as above, but with only 1 average to improve temporal resolution (1 image every 30 seconds).

Short-term pharmacokinetics acquired during the first 30 min were analyzed semi-quantitatively using the following expression:

$$RE = -\left|\frac{I_t - I_0}{I_0} \times 100\right|$$

where RE is the modulus of relative signal enhancement, It is the signal intensity at a given time after nanoparticle injection, and $I_0$ is the signal intensity before injection.

Long-term pharmacokinetics were measured by parametric (quantitative) $T_2$ imaging at 0 h, 0.5 h, 1 h, and 2 h. Pharmacokinetics were obtained by calculating the mean values within different regions of interest (ROI) in the following tissues: entire tumour and tumour periphery.

***Ex vivo* assays.**

Analysis of blood circulation times.

One hour after IONP injection, the mice were sacrificed and blood was collected for further analysis. Transmission Electron Microscopy (TEM) was used to determine the shape and size of PEGylated IONPs, and $^1H$ NMR relaxometry was used to measure the transverse relaxation rate ($R_2$) of blood plasma.

Histology.

The tissues were fixed in 4% formaldehyde (Panreac, buffered to pH 7) for 48 h, changing the 4% formaldehyde every 24 h. The samples were then dehydrated using graded ethanol and embedded in paraffin (temperature 56°C for 2 h under agitation and vacuum). The detailed procedures are described below.

Hematoxylin and eosin (H&E): Paraffin-embedded samples were sectioned at 7 μm thickness, deparaffinized, rehydrated, and stained with H&E, then dehydrated in ascending concentrations of ethanol, cleared in xylene, and mounted on commercial glass slides.

Prussian blue (PB): Paraffin-embedded samples were sectioned at 7 μm thickness, then deparaffinized, rehydrated, immersed in 20% hydrochloric acid and 10% potassium ferrocyanide, washed with water and counterstained with "Nuclear Fast Red", dehydrated in ascending concentrations of ethanol, cleared with xylene, and mounted on commercial glass slides.

**Results.**

In this work, the IONPs were functionalised with two different PEGylated ligands (OH and $NH_2$ terminations). In a subsequent chemical reaction, the NPs@$NH_2$ were covalently bound to glucuronic acid via an amide reaction. None of the chemical reactions affected the metal core.

Since the different functionalisation steps could affect the metal core of the IONPs, modifying their size and/or shape and, therefore, their magnetic properties, an exhaustive TEM analysis was necessary to ensure their stability **(Figure 1a).** In general, neither the shape nor the size of the IONPs were affected by the different synthetic steps, despite some aggregation in the case of NPs@$NH_2$. It should be noted that after covalent link with glucuronic acid, aggregation was no longer observed. Furthermore, the presence of the PEGylated ligand was confirmed by FTIR for NPs@OH and NPs@$NH_2$ **(Figure 1b),** indicating a favourable ligand exchange procedure. The covalent bond between NPs@$NH_2$ and glucuronic acid was also satisfactorily confirmed by FTIR. Furthermore, functionalisation with PEGylated ligands should increase colloidal stability in aqueous media. However, NPs@$NH_2$ showed very low colloidal stability, with precipitation visible to the naked eye after 48 hours **(Figure 1c).** In contrast, both NPs@OH and NPs@Gluc exhibited high colloidal stability, even after 500 hours.

The ability of these three IONPs as CAs for MRI was evaluated in two different magnetic fields: 1.44 T and 9.4 T **(Figure 2).** Both NPs@OH and NPs@Gluc exhibited excellent properties as CAs for MRI. Due to the low stability of NPS@$NH_2$, their $r_2$ could not be accurately determined.

With regard to cytotoxicity, neither NPs@OH **(Figure 3)** nor NPs@Gluc **(Figure 4)** exhibited cytotoxic effects on HFF-1 cells. No significant changes were observed in cell morphology, as observed by optical microscopy (**Figures 3a and 4a**), the total number of cells **(Figures 3d and 4d),** the percentage of dead cells **(Figures 3e and 4e),** or mitochondrial activity **(Figures 3f and 4f),** supporting the absence of cytotoxicity.

The targeting and cellular internalization capabilities of glucuronic acid-functionalised NPs were evaluated in both healthy (HFF-1) and tumour (C6, u87, and 4T1) cells. Cells were exposed to NPs@OH or NPs@Gluc **(Figure 5).** NPs@OH showed very little internalization in all cases, being only observable in C6 cells after 4 hours of incubation. In contrast, NPs@Gluc showed high internalization after 2 hours of exposure, mainly in C6 and 4T1 cells, and moderately lower in HFF-1 and u87 cells. The internalization of NPs@Gluc increases 4 hours post-exposure, particularly for C6 cells.

We investigated whether internalization of NPs@Gluc was mediated by specific recognition and interaction with glucose transporters (GLUTs), for which C6 cells were selected. First, the presence of GLUT1 and GLUT3, which have often been found to be overexpressed in a variety of tumour cells, was confirmed by Western blot analysis, showing high expression of both GLUTs, with GLUT3 being qualitatively higher, as expected **(Figure 6a).** Once the presence of GLUTs was confirmed, a glucose competition assay was performed to demonstrate their involvement in the transport of NPs@Gluc through the plasma membrane. The addition of glucose inhibited the entry of NPs@Gluc into C6 cells in a dose-dependent manner **(Figure 6b-c),** thus clearly demonstrating that this **process is mediated by GLUTs.**

These data demonstrate the biocompatibility of IONPs *in vitro,* as the results of these assays show no statistically significant variation compared to controls. Furthermore, the specific recognition and facilitated transport of NPs@Gluc by glucose transporters in different cell lines has been proven by the glucose competition assays performed.

However, tumour cell targeting *in vivo* is necessarily preceded by tumour targeting, i.e., the ability of NPs to accumulate selectively in the tumour tissue, which requires passive or active crossing the tumour vasculature to reach its interstitium. Passive tumour targeting is particularly difficult in brain tumours, where the blood-brain barrier is a major limiting factor. Therefore, the alternative strategy is active targeting. Given that GLUT1 is overexpressed in the vasculature of GBMs **[15],** it was proposed to administer NPs@Gluc to GBMs through GLUT-mediated active targeting. Thus, the pharmacokinetics of NPs@Gluc and NPs@OH were monitored *in vivo* using MRI.

Short-term pharmacokinetics **(Figure 7a)** exhibited a slight increase in signal after administration of PEGylated IONPs in groups G1 (9%) and G2 (11%), which remained constant from minute 10 until the end of the experiment. In contrast, in the case of G3 (24-hour fasting), there was a drastic increase in the signal (around 40%), indicating significant extravasation of NPs@Gluc into the tumour interstitium. Therefore, mild hypoglycemia promoted an outstanding increase in the amount of NPs@Gluc reaching the tumour. These data are consistent with *in vitro* glucose competition experiments, which showed much higher internalization of NPs@Gluc at lower glucose concentrations. In other words, decreasing glucose levels (by fasting or by using hypoglycemic agents) promotes efficient active transport of NPs@Gluc into the tumour interior mediated by GLUT. It should be noted that this increase in signal was observed both over the whole tumour and in the tumour periphery. As mentioned for G1 and G2, the signal remained constant after 10 minutes.

Regarding long-term pharmacokinetics (evaluated qualitatively using magnetic susceptibility-weighted images), no obvious changes were observed over time in the G1 group tumour, while the G2 group showed slight darkening 1 hour after NPs injection. In contrast, a very significant darkening was detected in the G3 group at that time. Tumours in groups G2 and G3 maintained a similar darkening 2 hours after injection. In conclusion, the 24-hour fasting period resulted in a decrease in blood glucose concentration, leading to a drastic increase in the accumulation of NPS@Gluc within the tumour. These results were also evaluated using quantitative MRI ($T_2$ maps). A statistically significant increase in $R_2$ of

approximately 2 and 5 $s^{-1}$ (for the whole tumour and tumour periphery, respectively) was observed 1 hour after NPs injection in group G3 **(Figure 7c-d).** These values were similar 2 hours after NPs injection. It should be noted that no changes in $R_2$ were observed in any case in the contralateral part of the brain, indicating selective accumulation in the tumour.

In order to increase tumour targeting, NPs must have a reasonably long residence time in blood. Therefore, blood was analyzed one hour after NPs injection using TEM and TD-NMR **(Figure 8).** A significant amount of both NPs@OH and NPs@Gluc continued to circulate in the bloodstream, exhibiting in all cases a relative increase in $R_2$ between 7 and 10 $s^{-1}$, which demonstrates the high stealth properties of these PEGylated NPs with respect to the animal's immune system.

In addition, to evaluate the accumulation of NPs in tissues, histological sections of the main organs were stained with Prussian blue **(Figure 9).** At the end of the experiment (2 hours after NPs injection), no signs of NPs accumulation were observed in either the tumour or the liver of the control mice (injected with PBS). On the other hand, the Kupffer cells of mice injected with both NPs@OH and NPs@Gluc showed a clear accumulation of NPs. As for the tumour, mice injected with NPs@OH showed low accumulation, while those injected with NPs@Gluc showed a significantly higher amount. In addition, NPs@Gluc were distributed more homogenously throughout the tumour tissue, which is also crucial for the effectiveness of tumour treatment.

Based on the above, it can be concluded that NPs@Gluc have an extraordinary capacity for tumour targeting *in vivo.* Specifically, NPs@Gluc were able to overcome the restrictions imposed by BBTB and effectively reach and accumulate in high-grade gliomas. Mild fasting-induced hypoglycemia greatly enhanced this ability, indicating that the observed vascular translocation of NPs@Gluc is likely mediated by GLUT1, which is known to be overexpressed in the vasculature of these tumours.

References

1. Sivasubramanian et al. Seeing Better and Going Deeper in Cancer Nanotheranostics. International Journal of Molecular Sciences. 2019; 20(14):3490.

2. Israel LL et al. Magnetic iron oxide nanoparticles for imaging, targeting and treatment of primary and metastatic tumours of the brain. J Control Release. 2020 Apr 10;320:45-62.

3. Nakamura Y. et al. Nanodrug Delivery: Is the Enhanced Permeability and Retention Effect Sufficient for Curing Cancer? Bioconjug Chem. 2016 Oct 19;27(10):2225-2238.

4. Caro C et al. D. Polysaccharide Colloids as Smart Vehicles in Cancer Therapy. Curr Pharm Des. 2015;21(33):4822-36. doi: 10.2174/1381612821666150820100812.

5. C. Caro et al., Passive targeting of high-grade gliomas via the EPR effect: a closed path for metallic nanoparticles? Biomaterials Sciences 9 (2021) 7984-7995.

6. B. Thiesen, et al., Clinical applications of magnetic nanoparticles for hyperthermia International Journal of Hyperthermia 24 (2008) 467-474.

7. T. Nishioka, et al., Distribution of the glucose transporters in human brain tumours. Cancer Research 52 (1992) 3972-3979.

8. F. Pistollato, et al., Intratumoural hypoxic gradient drives stem cells distribution and MGMT expression in glioblastoma. Stem Cells 28 (2010) 851-862.

9. M. R. Guda, et al., GLUT1 and TUBB4 in Glioblastoma Could be Efficacious Targets. Cancers 11(9) (2019) 1308.

10. K. Suzuki, et al., Glucose transporter 1-mediated vascular translocation of nanomedicines enhances accumulation and efficacy in solid tumours. Journal of Controlled Release 301 (2019) 28-41.

11. Singh, I., Swami, R., Jeengar, M.K., Khan, W., Sistla, R., 2015. p-Aminophenyl-$\alpha$-d-mannopyranoside engineered lipidic nanoparticles for effective delivery of docetaxel to brain. Chem. Phys. Lipids 188, 1-9. https://doi.org/10.1016/j chemphyslip.2015.03.003.

12. Sawayama, H. & Baba, H. in Molecular Nutrition: Carbohydrates (ed Vinood B. Patel) 191-207 (Academic Press, 2019). https://doi.org/https://doi.org/10.1016/B978-0-12-849886-6.00007-0.

13. A. Harland, et al., Glioma Stem-Like Cells and Metabolism: Potential for Novel Therapeutic Strategies. Frontiers in Oncology 11 (2021) 743814.

14. Blazek, et al., Int J Radiat Oncol Biol Phys 67(1) (2007) 1-5. doi:10.1016/j.ijrobp.2006.09.037; Liu, et al., Mol Cancer 5 (2006) 67. doi:10.1186/1476-4598-5-67.]

15. W.-L. Yeh, et al., Enhancement of glucose transporter expression of brain endothelial cells by vascular endothelial growth factor derived from glioma exposed to hypoxia Mol Pharmacol. 73(1) (2008) 170-177.

## Claims

1. Metallic magnetic nanoparticle whose metallic core is composed of iron oxide comprising at least one organic ligand composed of a gallic acid bound to a polyethylene glycol derivative attached to its surface, **characterised in that** it is functionalised with at least one molecule of a glucose derivative.

2. Metallic magnetic nanoparticle according to the previous claim **characterised in that** the glucose derivative is glucuronic acid.

3. Metallic magnetic nanoparticle according to the previous claim **characterised in that** the polyethylene glycol derivative is bis-amino-polyethylene glycol.

4. Method for synthesizing metallic magnetic nanoparticles according to any of the preceding claims **characterised in that** it comprises the following steps:

   a) Synthesis of the metal core from iron oleate or its derivatives, using a chemical or physical process selected from the list comprising evaporation, condensation, laser ablation, hydrothermal method, chemical reduction, irradiation, coprecipitation, electrochemistry, microemulsion, thermal decomposition, and photoreduction.
   b) Functionalisation with a polymeric ligand synthesized by a covalent bond between gallic acid and a polyethylene glycol or bis-amino-polyethylene glycol derivative by a physical or chemical method.
   c) Functionalisation with a glucose derivative or glucuronic acid.

5. Metallic magnetic nanoparticle according to any of claims 1 to 3 for use as a contrast agent for Magnetic Resonance Imaging.

6. Metallic magnetic nanoparticle according to the previous claim for use in a method of acquisition of images, evaluation, and/or diagnosis of solid tumours or metastatic lesions of the central nervous system.

7. Metallic magnetic nanoparticle according to any of claims 1 to 3 for use as a drug delivery platform.

8. Metallic magnetic nanoparticle according to the previous claim for use in a method of treating solid tumours or metastatic lesions of the central nervous system.

9. Metallic magnetic nanoparticle according to any of claims 1 to 3 for use as a magnetic hyperthermia mediator.

10. Metallic magnetic nanoparticle according to the preceding claim for use in a method of treating solid tumours or metastatic lesions of the central nervous system.

11. Metallic magnetic nanoparticle according to any of claims 1 to 3 for use via intratumoural, intranasal, intraperitoneal, intravenous, intraarterial, anal, or oral administration.

12. Metallic magnetic nanoparticle according to any of claims 1 to 3 for use according to any of claims 5 to 11 **characterised in that** the patient is in a state of mild hypoglycemia or close to the lower limit of preprandial normoglycemia.

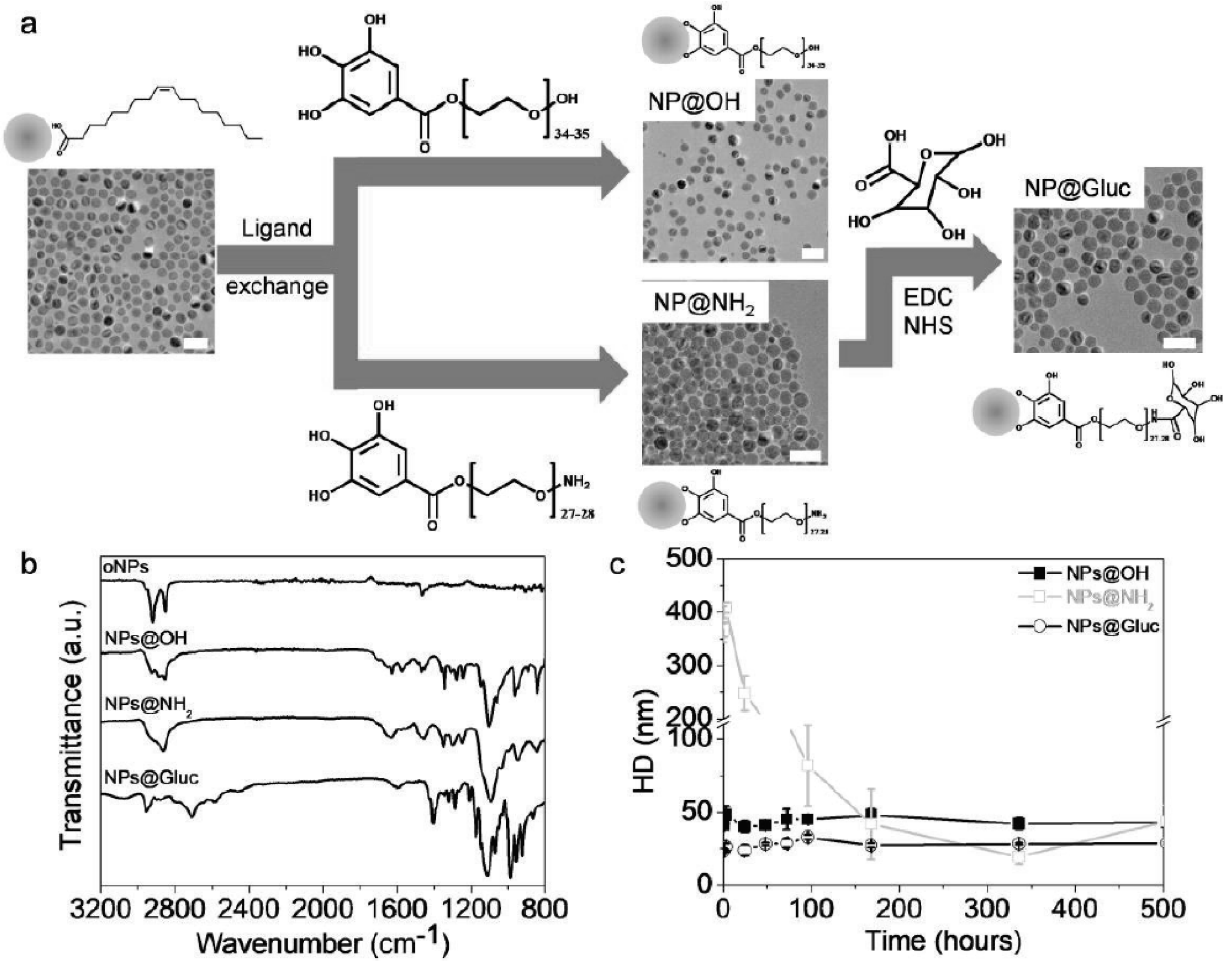
a
NP@OH
Ligand
exchange
NP@NH2
EDC
NHS
NP@Gluc
b
oNPs
NPs@OH
NPs@NH2
NPs@Gluc
Transmittance (a.u.)
3200 2800 2400 2000 1600 1200 800
Wavenumber (cm-1)
c
NPs@OH
NPs@NH2
NPs@Gluc
HD (nm)
Time (hours)

FIG.1

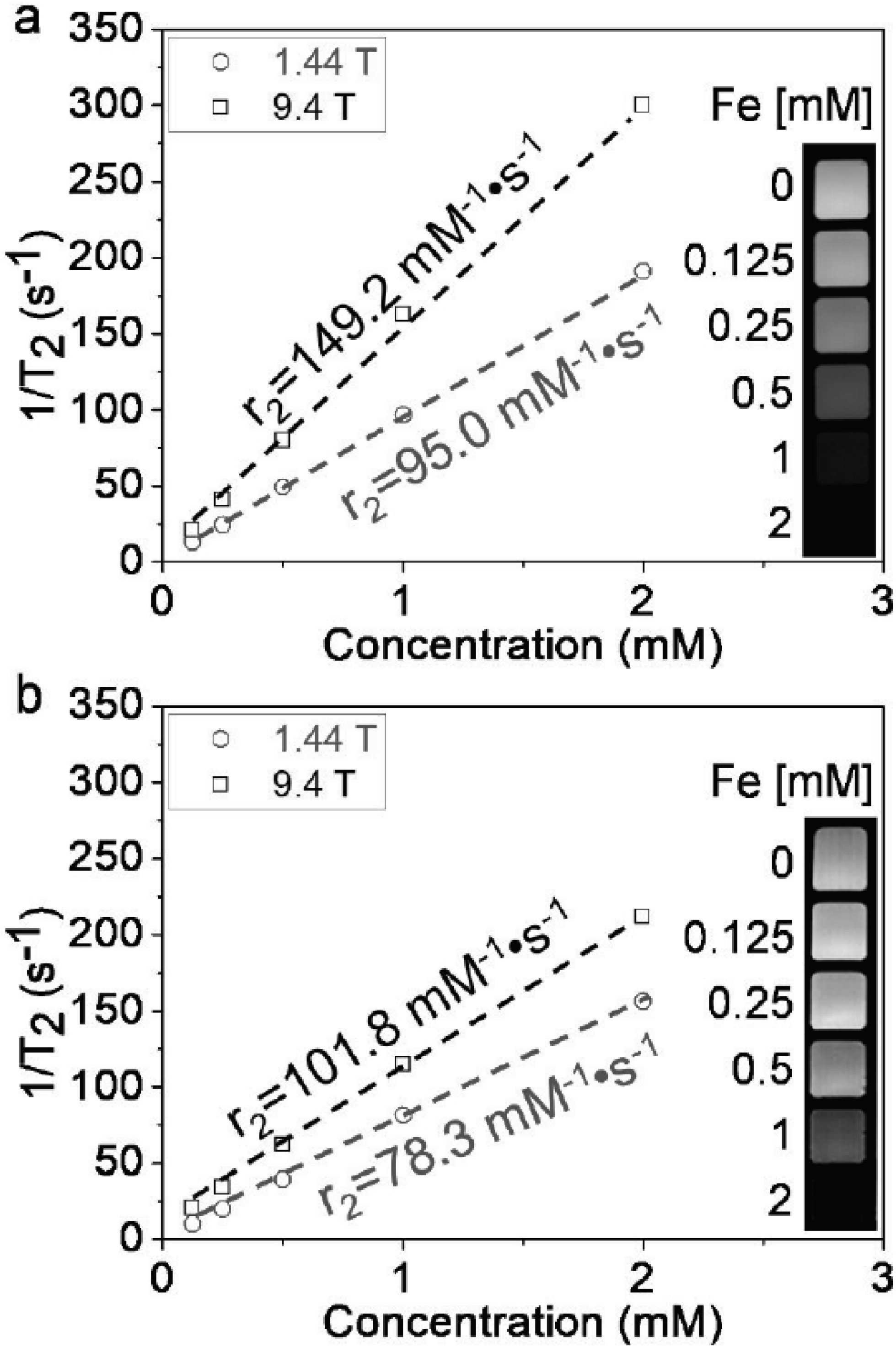
a
1.44 T
9.4 T
Fe [mM]
0
0.125
0.25
0.5
1
2
r2=149.2 mM-1•s-1
r2=95.0 mM-1•s-1
1/T2 (s-1)
Concentration (mM)
b
1.44 T
9.4 T
Fe [mM]
0
0.125
0.25
0.5
1
2
r2=101.8 mM-1•s-1
r2=78.3 mM-1•s-1
1/T2 (s-1)
Concentration (mM)

FIG.2

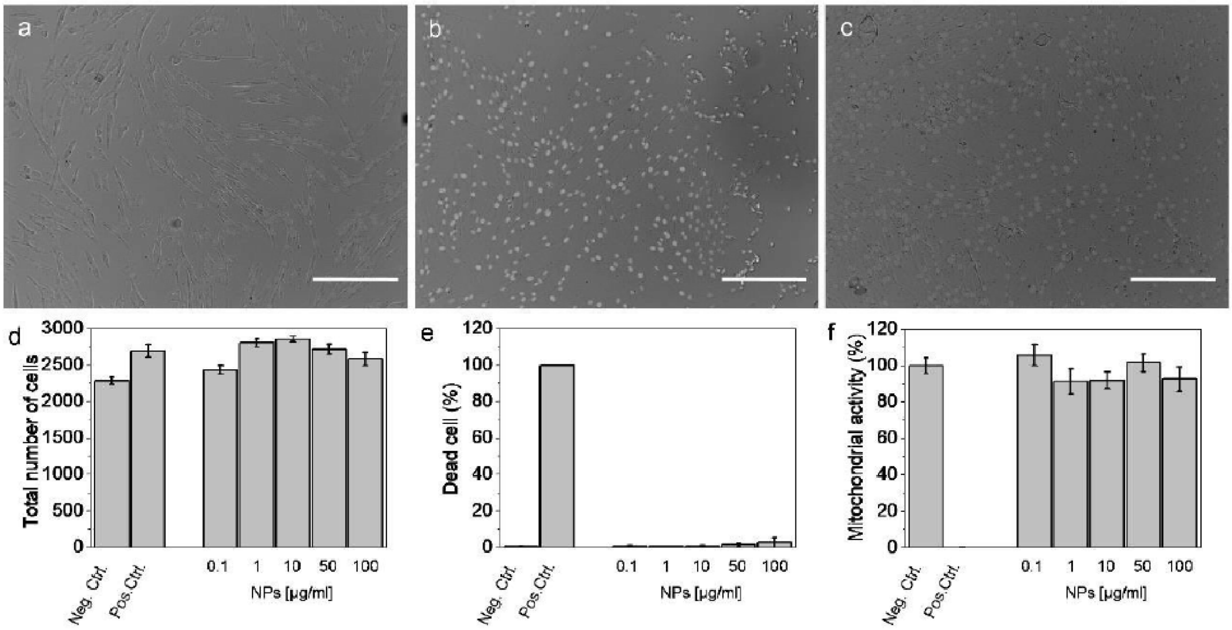
a
b
c
d
Total number of cells
3000
2500
2000
1500
1000
500
0
Neg. Ctrl.
Pos.Ctrl.
0.1 1 10 50 100
NPs [µg/ml]
e
Dead cell (%)
120
100
80
60
40
20
0
Neg. Ctrl.
Pos.Ctrl.
0.1 1 10 50 100
NPs [µg/ml]
f
Mitochondrial activity (%)
120
100
80
60
40
20
0
Neg. Ctrl.
Pos.Ctrl.
0.1 1 10 50 100
NPs [µg/ml]

FIG.3

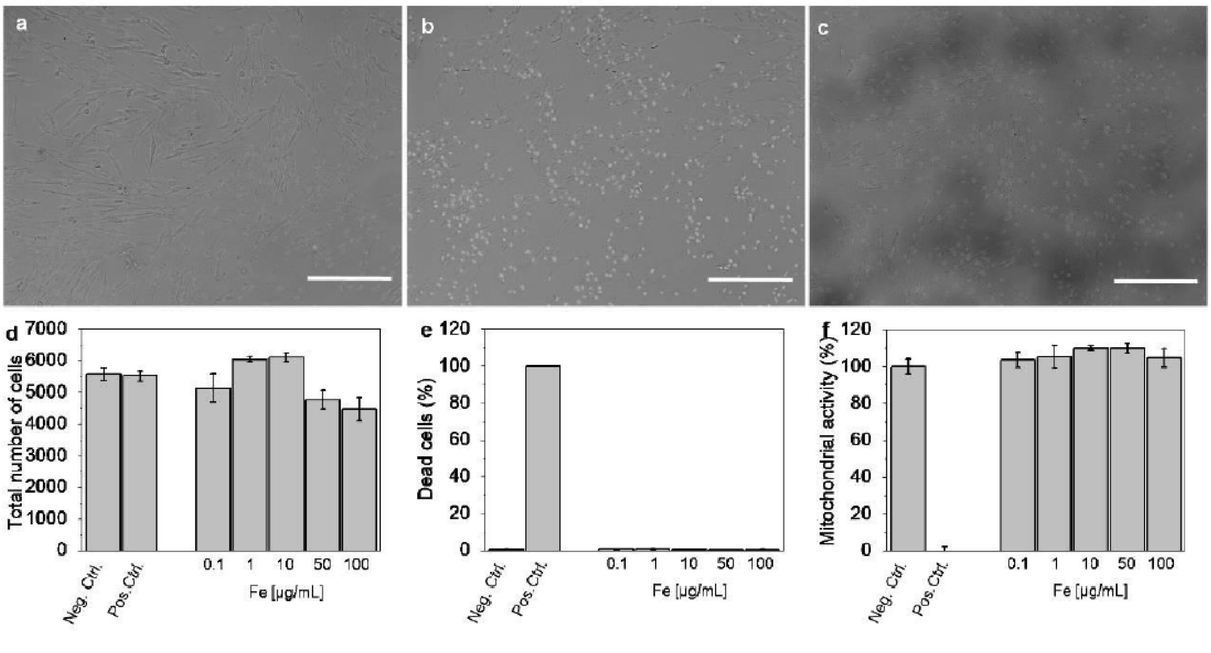
a
b
c
d
Total number of cells
7000
6000
5000
4000
3000
2000
1000
0
Neg. Ctrl.
Pos.Ctrl.
0.1 1 10 50 100
Fe [µg/mL]
e
Dead cells (%)
120
100
80
60
40
20
0
Neg. Ctrl.
Pos.Ctrl.
0.1 1 10 50 100
Fe [µg/mL]
f
Mitochondrial activity (%)
120
100
80
60
40
20
0
Neg. Ctrl.
Pos.Ctrl.
0.1 1 10 50 100
Fe [µg/mL]

FIG.4

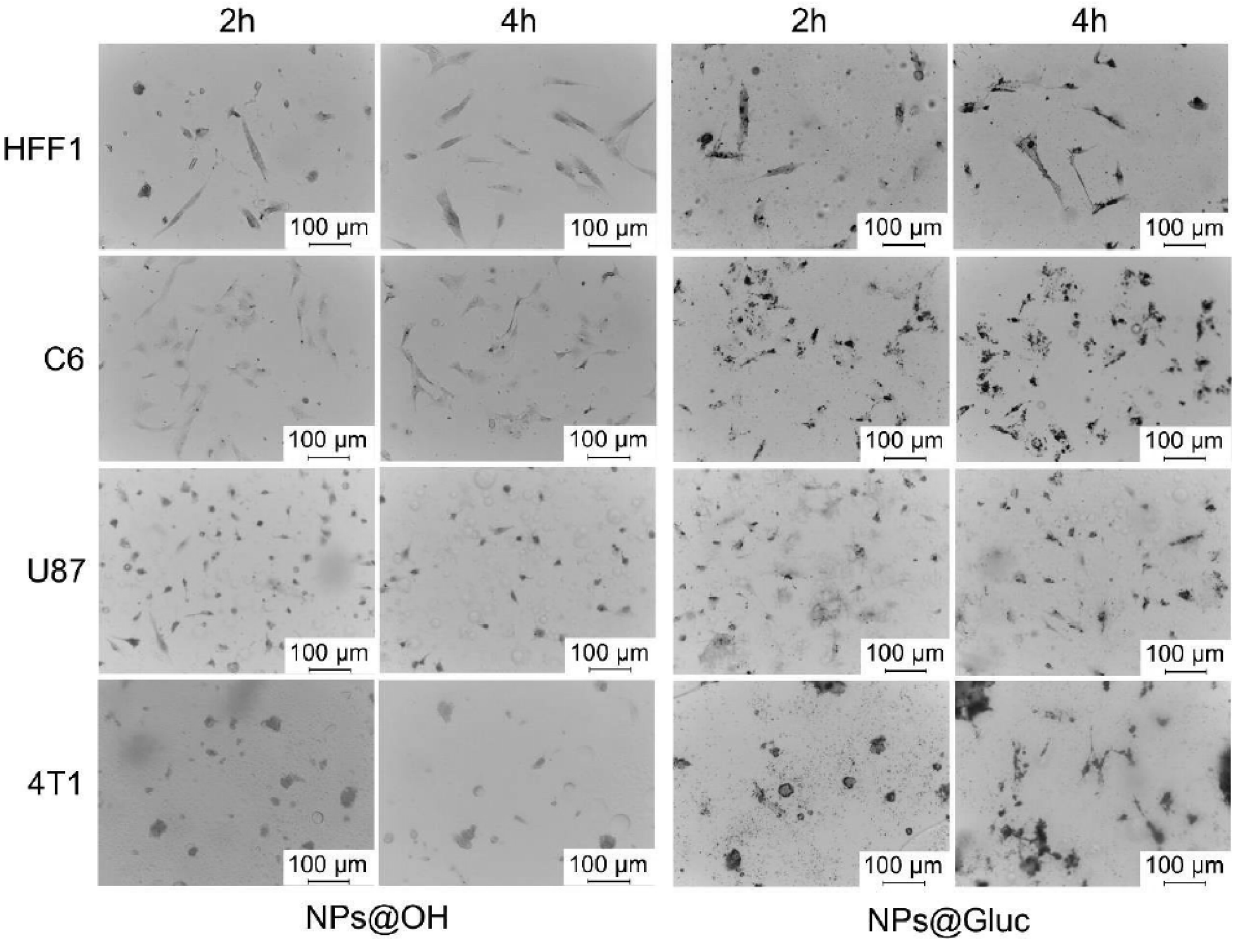


FIG.5

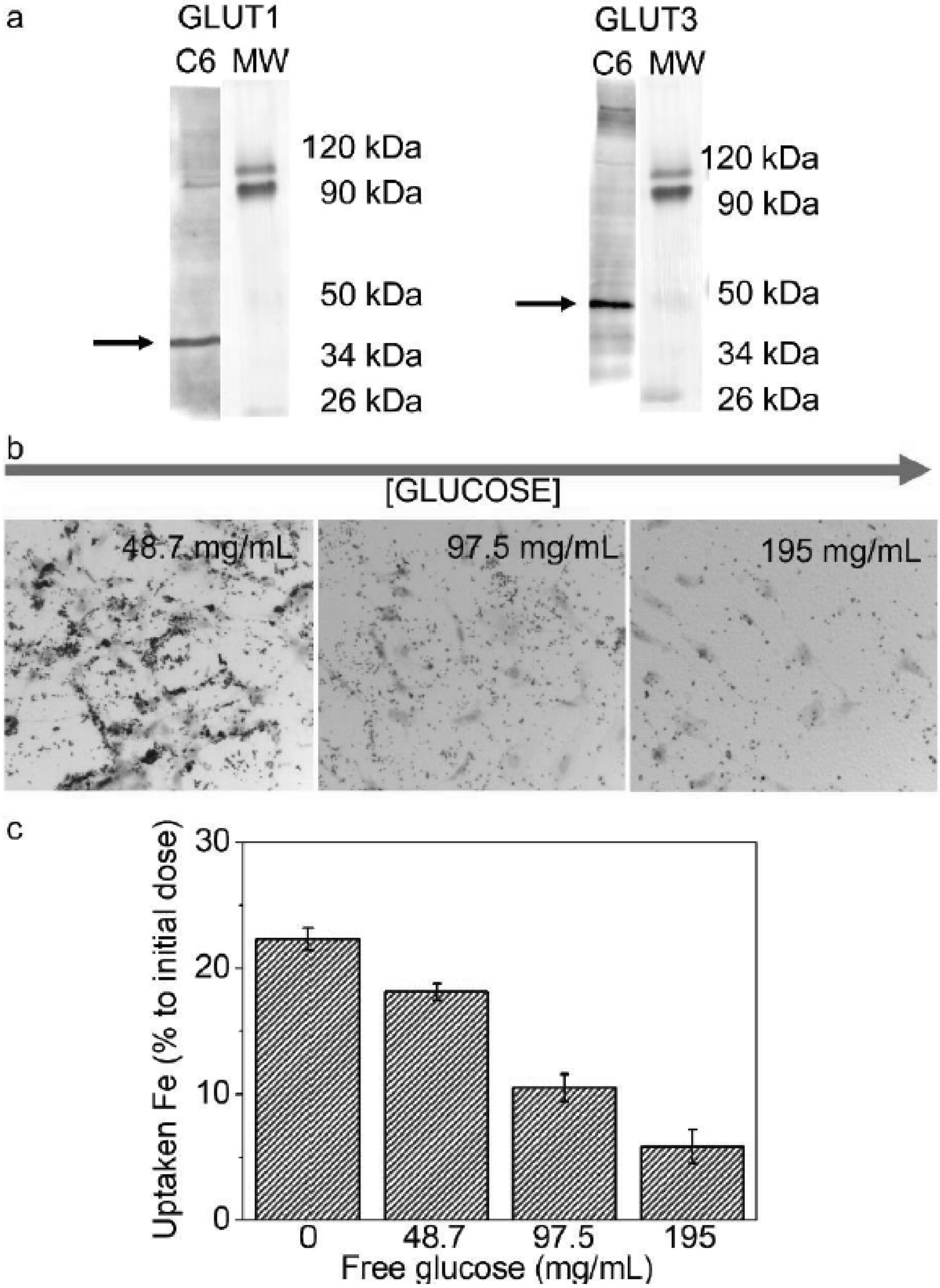
a
GLUT1
C6 MW
120 kDa
90 kDa
50 kDa
34 kDa
26 kDa
GLUT3
C6 MW
120 kDa
90 kDa
50 kDa
34 kDa
26 kDa
b
[GLUCOSE]
48.7 mg/mL
97.5 mg/mL
195 mg/mL
c
Uptaken Fe (% to initial dose)
30
20
10
0
0
48.7
97.5
195
Free glucose (mg/mL)

FIG.6

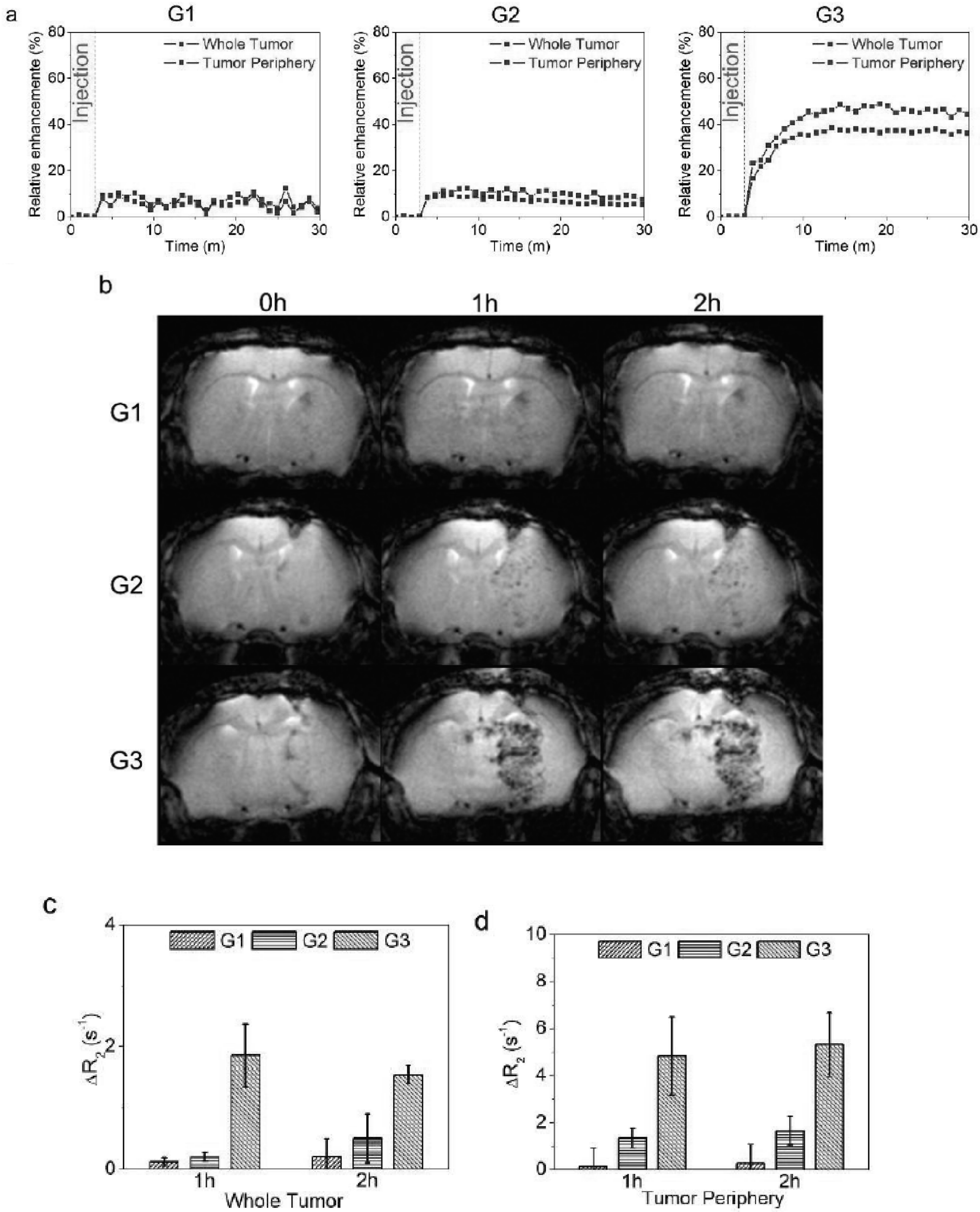
a
G1
G2
G3
Injection
— Whole Tumor
— Tumor Periphery
Relative enhancemente (%)
Time (m)
b
0h
1h
2h
G1
G2
G3
c
d
G1
G2
G3
ΔR2 (s-1)
1h
2h
Whole Tumor
Tumor Periphery

FIG.7

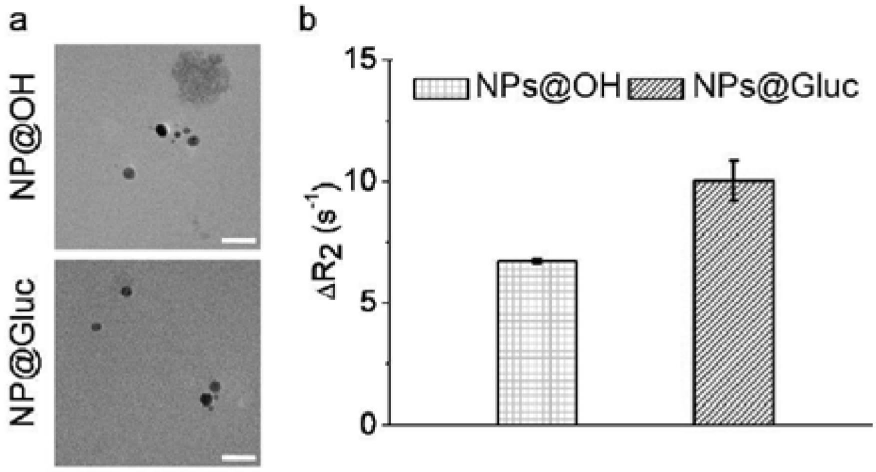
a
NP@OH
NP@Gluc
b
NPs@OH
NPs@Gluc
ΔR2 (s-1)
15
10
5
0

FIG.8

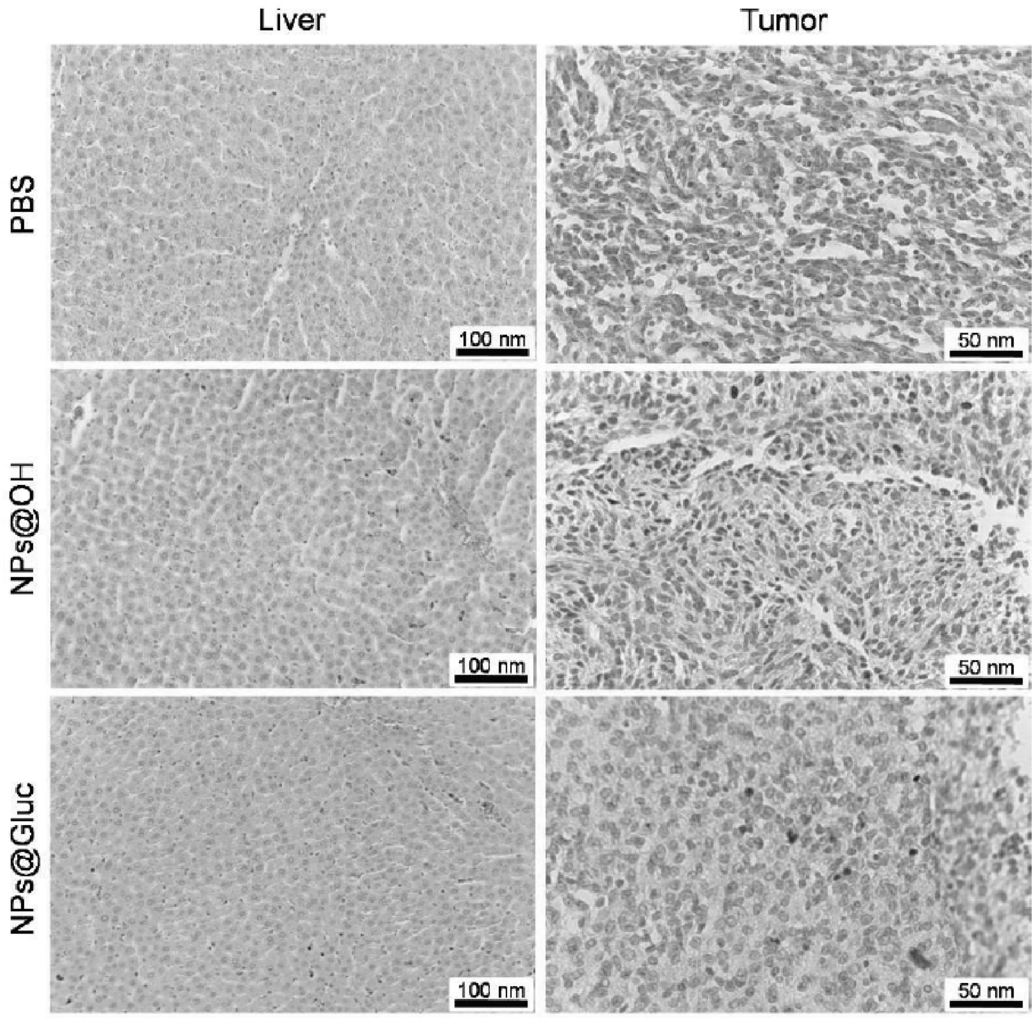
Liver
Tumor
PBS
NPs@OH
NPs@Gluc
100 nm
50 nm

FIG.9

## INTERNATIONAL SEARCH REPORT

International application No.
PCT/ES2024/070474

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)
A61K, B82Y, A61B, A61N

Documentation searched other than minimum documentation to the extent that such documents are included in the fields searched

Electronic data base consulted during the international search (name of data base and, where practicable, search terms used)

EPODOC, INVENES, NPL, INTERNET

C. DOCUMENTS CONSIDERED TO BE RELEVANT

| Category* | Citation of document, with indication, where appropriate, of the relevant passages | Relevant to claim No. |
|---|---|---|
| A | LEAL, M. P. et al.: “In vivo pharmacokinetics of T2 contrast agents based on iron oxide nanoparticles: Optimization of blood circulation times”. RSC Advances 20150830 Royal Society Of Chemistry GBR, 30/08/2015, Vol. 5, Nº 94, pages 76883 - 76891, ISSN 2046-2069 (electronic), <DOI: 10.1039/c5ra15680g>, pages 76883, 76889, 76890. | 1-12 |
| A | LUIZ MARCELA TAVARES et al.: “Highlights in targeted nanoparticles as a delivery strategy for glioma treatment”. International Journal Of Pharmaceutics, 20210604 Elsevier, Amsterdam, NL, 04/06/2021, Vol. 604, ISSN 0378-5173, <DOI: 10.1016/j.ijpharm.2021.120758>. | 1-12 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 05/12/2024 | **(10/12/2024)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>S. Sánchez Paradinas<br><br>Telephone No. 913493280 |

Form PCT/ISA/210 (second sheet) (July 2022)

**INTERNATIONAL SEARCH REPORT**

International application No.
PCT/ES2024/070474

C (continuation). DOCUMENTS CONSIDERED TO BE RELEVANT

| Category * | Citation of documents, with indication, where appropriate, of the relevant passages | Relevant to claim No. |
|---|---|---|
| A | UTHAMAN, S. et al.: "Polysaccharide-coated magnetic nanoparticles for imaging and gene therapy". Biomed Research International 2015, Hindawi Publishing Corporation USA, 30/11/2014, Vol. 2015, ISSN 2314-6133 (print) ISSN 2314-6141 (electronic), <DOI: 10.1155/2015/959175 pubmed:26078971>. | 1-12 |
| A | WO 2009136763 A2 (IND ACADEMIC COOP ET AL.) 12/11/2009. | 1-12 |

Form PCT/ISA/210 (continuation of second sheet) (July 2022)

## INTERNATIONAL SEARCH REPORT

Information on patent family members

International application No.
PCT/ES2024/070474

| Patent document cited in the search report | Publication date | Patent family member(s) | Publication date |
|---|---|---|---|
| WO2009136763 A2 | 12.11.2009 | KR20090117648 A<br>KR101081445B B1 | 12.11.2009<br>08.11.2011 |

Form PCT/ISA/210 (patent family annex) (July 2022)

**INTERNATIONAL SEARCH REPORT**

International application No.

PCT/ES2024/070474

CLASSIFICATION OF SUBJECT MATTER

***A61K49/18*** (2006.01)
***B82Y5/00*** (2011.01)
***B82Y15/00*** (2011.01)
*A61K41/00* (2020.01)
*A61B5/055* (2006.01)
*A61N2/00* (2006.01)

Form PCT/ISA/210 (extra sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SIVASUBRAMANIAN et al.** Seeing Better and Going Deeper in Cancer Nanotheranostics.. *International Journal of Molecular Sciences.*, 2019, vol. 20 (14), 3490
- **ISRAEL LL et al.** Magnetic iron oxide nanoparticles for imaging, targeting and treatment of primary and metastatic tumours of the brain.. *J Control Release.*, 10 April 2020, vol. 320, 45-62
- **NAKAMURA Y. et al.** Nanodrug Delivery: Is the Enhanced Permeability and Retention Effect Sufficient for Curing Cancer. *Bioconjug Chem.*, 19 October 2016, vol. 27 (10), 2225-2238
- **CARO C et al.** D. Polysaccharide Colloids as Smart Vehicles in Cancer Therapy.. *Curr Pharm Des.*, 2015, vol. 21 (33), 4822-36
- **C. CARO et al.** Passive targeting of high-grade gliomas via the EPR effect: a closed path for metallic nanoparticles. *Biomaterials Sciences*, 2021, vol. 9, 7984-7995
- **B. THIESEN et al.** *Clinical applications of magnetic nanoparticles for hyperthermia International Journal of Hyperthermia*, 2008, vol. 24, 467-474
- **T. NISHIOKA et al.** Distribution of the glucose transporters in human brain tumours.. *Cancer Research*, 1992, vol. 52, 3972-3979
- **F. PISTOLLATO et al.** Intratumoural hypoxic gradient drives stem cells distribution and MGMT expression in glioblastoma.. *Stem Cells*, 2010, vol. 28, 851-862
- **M. R. GUDA et al.** GLUT1 and TUBB4 in Glioblastoma Could be Efficacious Targets. *Cancers*, 2019, vol. 11 (9), 1308
- **K. SUZUKI et al.** Glucose transporter 1-mediated vascular translocation of nanomedicines enhances accumulation and efficacy in solid tumours.. *Journal of Controlled Release*, 2019, vol. 301, 28-41
- **SINGH, I** ; **SWAMI, R** ; **JEENGAR, M.K** ; **KHAN, W** ; **SISTLA, R.** p-Aminophenyl-α-d-mannopyranoside engineered lipidic nanoparticles for effective delivery of docetaxel to brain.. *Chem. Phys. Lipids*, 2015, vol. 188, 1-9, https://doi.org/10.1016/j chemphyslip.2015.03.003.
- **SAWAYAMA, H.** ; **BABA, H.** Molecular Nutrition: Carbohydrates. Academic Press, 2019, 191-207
- **A. HARLAND et al.** Glioma Stem-Like Cells and Metabolism: Potential for Novel Therapeutic Strategies.. *Frontiers in Oncology*, 2021, vol. 11, 743814
- **BLAZEK et al.** *Int J Radiat Oncol Biol Phys*, 2007, vol. 67 (1), 1-5
- **LIU et al.** *Mol Cancer*, 2006, vol. 5, 67
- **W.-L. YEH et al.** Enhancement of glucose transporter expression of brain endothelial cells by vascular endothelial growth factor derived from glioma exposed to hypoxia. *Mol Pharmacol.*, 2008, vol. 73 (1), 170-177